# FASCICULE DE BREVET EUROPEEN

(11) **EP 3 573 457 B1**
(45) Date de publication et mention de la délivrance du brevet: **10.12.2025**
(21) Numéro de dépôt: 18705436.6
(22) Date de dépôt: 30.01.2018
(51) Int. Cl.: A01N 1/145, A01N 1/162, A01N 1/165

(54) **PROCÉDÉ DE REFROIDISSEMENT D'UNE MATIÈRE BIOLOGIQUE ET SA CONSERVATION**
VERFAHREN ZUR KÜHLUNG EINES BIOLOGISCHEN MATERIALS UND ZUR LAGERUNG DAVON
PROCESS FOR COOLING A BIOLOGICAL MATERIAL AND THE STORAGE THEREOF

(30) Priorité: 30.01.2017 FR 1750745
(43) Date de publication de la demande: 04.12.2019
(73) Titulaire: Genialis, 18250 Henrichemont (FR)
(72) Inventeur: DESJARDINS, Isabelle, 18250 Henrichemont (FR); GILLET, Guillaume, 41300 Salbris (FR)
(74) Mandataire: Decobert, Ludivine Marie
(86) Numéro de dépôt international: PCT/FR2018/050208
(87) Numéro de publication internationale: WO 2018/138461

(56) Documents cités:
- EP-A1- 0 232 672
- WO-A1-99/66271
- WO-A2-2007/123720
- DE-A1- 102011 115 467
- US-A1- 2009 011 505
- KATSUHIDE OHIRA ET AL: "Numerical study of flow and heat-transfer characteristics of cryogenic slush fluid in a horizontal circular pipe (SLUSH-3D)", CRYOGENICS, ELSEVIER, KIDLINGTON, GB, vol. 52, no. 7, 21 April 2012 (2012-04-21), pages 428 - 440, XP028513157, ISSN: 0011-2275, [retrieved on 20120430], DOI: 10.1016/J.CRYOGENICS.2012.04.006
- PRIBENSZKY C ET AL: "Improving post-thaw survival of cryopreserved mouse blastocysts by hydrostatic pressure challenge", ANIMAL REPRODUCTION SCIENCE, ELSEVIER SCIENCE PUBLISHERS, AMSTERDAM, NL, vol. 87, no. 1-2, 1 June 2005 (2005-06-01), pages 143 - 150, XP027622634, ISSN: 0378-4320, [retrieved on 20050601]

## Description

### Domaine technique

La présente invention concerne le traitement de matières biologiques en vue d'assurer leur conservation dans le temps. En particulier, elle concerne la conservation de matières biologiques par cryogénie sous pression.

### État de la technique

Le principe de la cryogénie est la soumission d'une matière biologique au froid. Les premiers essais ont débuté dans les années 1950 avec la conservation de sperme humain et bovin qui est une matière biologique en suspension. Une température est dite cryogénique quand elle est inférieure à 93,15 K selon une définition de l'US National Institute of Standards and Technology (institut national américain de normalisation et des technologies). Toutefois, par extension, les applications utilisant l'immersion dans des gaz liquides jusqu'à 170 K, voire utilisant la pulvérisation de tels gaz jusqu'à 230 K, tunnels cryogéniques IQF (Individually Quick Frozen, en français « surgélation séparée ») par exemple, sont considérées comme des applications cryogéniques. L'idée étant qu'à basse température, environ 190 K, les activités chimiques se produisant normalement à l'intérieur de la matière biologique cessent et en dessous de 140 K, tous les corps non cristallins sont vitreux et donc complètement stabilisés. Ainsi, les processus de métabolisme, de vieillissement et de mort n'ont pas lieu, ce qui permet à la matière biologique de rester intacte dans un état congelé.

La cryogénie a ensuite été adaptée pour la conservation d'autres matières biologiques telles que les composants du sang humain (par exemple les érythrocytes).

Dans les années 1960, des procédés chirurgicaux de transplantation d'organes ont été développés. Cependant, les praticiens rencontrent trois problèmes majeurs : le rejet par le patient de l'organe transplanté, la disponibilité d'un organe compatible avec le patient et la durée de vie limitée de l'organe en dehors du corps.

C'est pourquoi, la conservation par cryogénie a depuis de nombreuses années nourrit les espoirs des praticiens, mais surtout des patients en attente d'une transplantation afin de pouvoir conserver à long terme des organes prélevés la plupart du temps à la mort du donneur et ainsi disposer d'une banque d'organes résoudrait les trois problèmes mentionnés ci-dessus.

La difficulté de mise en œuvre des procédés de conservation par cryogénie ne réside pas dans la viabilité de la matière biologique dans des conditions extrêmes de température cryogénique, mais l'existence d'une zone de température intermédiaire entre la température ambiante et la température cryogénique létale pour celle-ci. Cette zone de température est comprise entre environ 220 K et environ 255 K. La matière biologique ne doit pas traverser cette zone seulement une fois, mais bien deux fois : une première fois pendant l'abaissement de la température jusqu'à une température cryogénique pour sa conservation et une deuxième fois pendant le réchauffage à la température ambiante pour son utilisation.

À cette température, l'eau contenue dans la matière biologique se transforme en glace tout en augmentant de volume (d'environ 10%) : les cristaux de glace formés à l'extérieur des cellules sont de grande dimension et leur forme provoque la perforation des membranes cellulaires.

Si le refroidissement est lent, c'est l'eau à l'extérieur des cellules qui a tendance à se transformer en glace, ce qui provoque l'osmose de l'eau, la déplaçant de l'intérieur des cellules vers l'extérieur de celles-ci. Lorsque l'eau s'échappe des cellules, les solutés compris à l'intérieur de celles-ci voient leur concentration dangereusement augmenter jusqu'à en devenir létale.

Afin d'éviter la formation de glace, certains procédés de conservation par cryogénie emploient des actifs cryoprotecteurs, typiquement du glycérol, du diméthylsulfoxyde, des alcènes glycol ou autres composés capables de se lier fortement avec l'eau par liaison hydrogène. Cette capacité permet d'empêcher l'eau de geler en abaissant la température de solidification. Plus la quantité d'actifs cryoprotecteurs est importante et plus la température de solidification est abaissée.

Cependant, les actifs cryoprotecteurs ne sont généralement efficaces que pour la conservation de la matière biologique en suspension sous forme de cellules individuellement dissociées.

Par ailleurs, l'utilisation d'actifs cryoprotecteurs n'est pas sans danger pour la matière biologique. En effet, à haute concentration, l'actif cryoprotecteur devient lui-même nuisible à la matière biologique et contribue à la mortalité de celle-ci.

En outre, existe le problème de la suppression de l'actif cryoprotecteur avant l'utilisation de la matière biologique.

Enfin, même en présente d'actifs cryoprotecteurs, la survie à la conservation par cryogénie des cellules de la matière biologique est faible.

D'autres procédés font intervenir la pression afin de faire baisser la température de transformation de l'eau en glace. Par exemple, les travaux de thèse Tony Vien Le Bui (Cryopreservation, culture recovery and glucose induced programmed cell death in chlorophyte microalgae, The University of Queensland, 2014 ; « Cryopréservation, récupération de culture et mort programmée de cellules induite par glucose dans les microalgues chrolophytes » en français) abordent la question de la cryogénie sous pression en utilisant du matériel conçu notamment pour la microscopie électronique. Ces travaux rapportent des taux de survie ne dépassant pas 1%.
WO99/66271 A1 décrit un appareil d'injection directe comprenant un réceptacle contenant du réfrigérant sous forme de slush. Un cylindre avec de petites ouvertures d'injection est positionné dans le réceptacle de telle sorte que les ouvertures d'injection sont immergées dans le réfrigérant. Un échantillon sous pression est délivré à travers les ouvertures d'injection dans le réfrigérant. L'utilisation de haute pression dans ce procédé est également suggérée.

Ainsi, le besoin d'un procédé pour la conservation de matières biologiques avec un haut taux de survie se fait encore sentir.

### Présentation de l'invention

Un objectif de la présente invention est de pallier au moins un des inconvénients de l'état de la technique décrit ci-dessus et notamment de permettre la conservation de matières biologiques avec un haut taux de survie et notamment sans utilisation d'actifs cryoprotecteurs.

Pour cela, la présente invention propose un procédé de refroidissement de matière biologique par cryogénie sous pression, comprenant la solidification de la matière biologique par un fluide cryogénique, dans lequel la solidification de la matière biologique s'effectue à une température cryogénique, à une pression de 10 bars à 1000 bars et le fluide cryogénique est présent dans une quantité supérieure à 200 kg/m³.

Le réglage conjoint à la fois de la température cryogénique, de la pression et de la quantité de fluide cryogénique permet d'améliorer sensiblement le taux de survie des cellules de la matière biologique à conserver. En effet, les présents inventeurs ont découvert de manière surprenante que la température et la pression ne sont pas les seuls paramètres permettant de garantir un bon taux de survie, la prise en compte de la quantité de fluide cryogénique est tout aussi primordiale. Le fluide cryogénique est sous forme liquide ou supercritique.

Le procédé comprend en outre avant l'étape de solidification l'introduction de la matière biologique dans une enceinte contenant un fluide cryogénique ; et le réglage de conditions souhaitées de température, pression et quantité de fluide cryogénique à l'intérieur de l'enceinte à une température cryogénique, de sorte que la pression régnant à l'intérieur de l'enceinte soit de 10 bars à 1000 bars et que la quantité de fluide cryogénique contenue dans l'enceinte soit supérieure à 200 kg/m³. D'autres caractéristiques optionnelles et non limitatives sont les suivantes.

La température est de préférence réglée de manière à être inférieure à 170 K.

La pression est de préférence réglée de manière à être supérieure à 20 bars.

La quantité de fluide cryogénique contenu dans l'enceinte est de préférence réglée de manière à être supérieure à 250 kg/m³.

La matière biologique peut être au préalable contenue en suspension dans un mélange liquide ; le réglage de conditions souhaitées réalisé avant l'introduction de la matière biologique à l'intérieur de l'enceinte ; et l'introduction de la matière biologique réalisée par injection du mélange liquide à l'intérieur de l'enceinte.

L'enceinte peut comprendre une première partie d'enceinte et une deuxième partie d'enceinte, les volumes internes des première et deuxième parties d'enceinte étant au départ séparés ; l'étape d'introduction comprenant la disposition de la matière biologique dans la première partie d'enceinte exempte de fluide cryogénique, la disposition du fluide cryogénique disposé dans la deuxième partie d'enceinte et la réunion de la première partie d'enceinte avec la deuxième partie d'enceinte pour former l'enceinte de sorte que leurs volumes internes soient liés ; le réglage des conditions souhaitées étant réalisé dans la deuxième partie d'enceinte de sorte à obtenir les conditions souhaitées dans l'enceinte après la réunion de la première partie d'enceinte avec la deuxième partie d'enceinte.

L'invention propose également un procédé de conservation de matière biologique par cryogénie sous haute pression, comprenant les étapes du procédé décrit ci-dessus, la dépressurisation de l'enceinte à la pression atmosphérique et la disposition de la matière biologique solidifiée dans un congélateur.

La présente divulgation propose enfin un produit congelé comprenant de la matière biologique congelée préalablement traitée et présentant un taux de survie après décongélation de la matière biologique d'au moins 45%.

### Description des dessins

D'autres objectifs, caractéristiques et avantages apparaitront à la lecture de la description qui suit en référence aux dessins parmi lesquels :
- la figure 1 est un organigramme représentant les étapes du procédé de refroidissement d'une matière biologique selon un exemple de l'invention ;
- la figure 2 est un organigramme représentant les étapes du procédé de refroidissement d'une matière biologique selon un autre exemple de l'invention ;
- la figure 3 est un organigramme montrant les étapes d'un procédé de conservation ainsi que la décongélation d'une matière biologique selon l'invention ; et
- la figure 4 représente une photographie de spiruline conservée selon l'exemple 4 et prélevée après 2 semaines au congélateur à -20°C.

La description qui suit ainsi que les dessins sont donnés à titre purement illustratif et non limitatif.

### Description détaillée

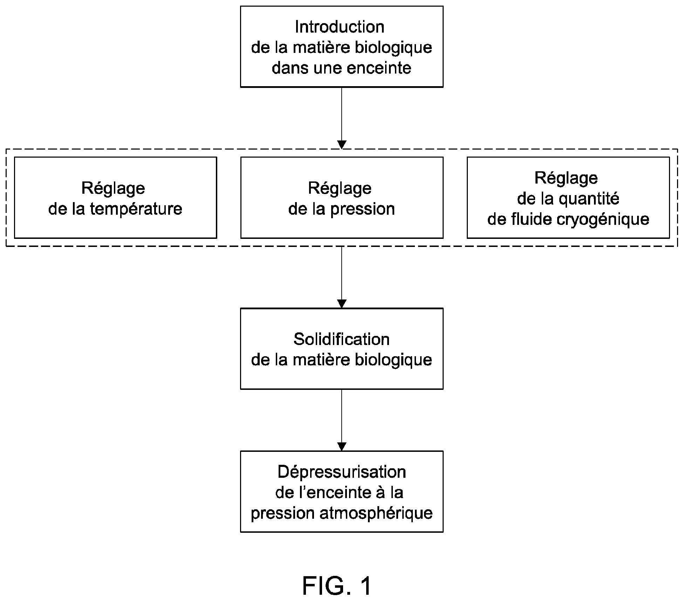

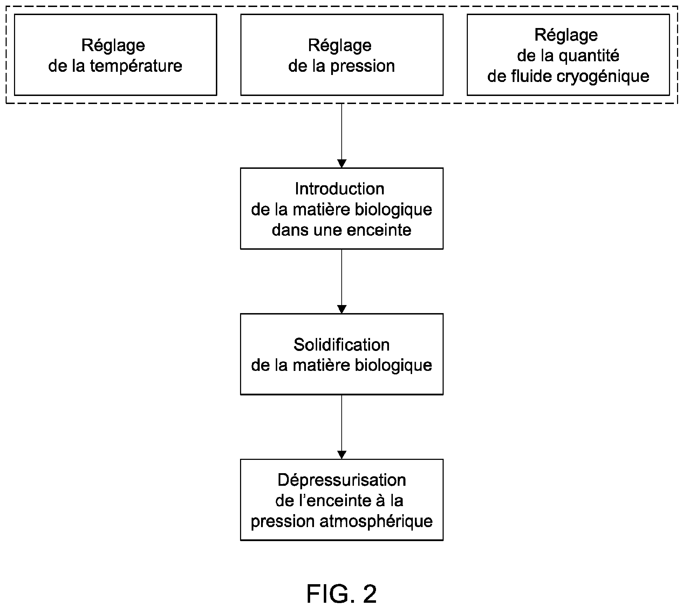

Un procédé de refroidissement de matière biologique par cryogénie selon la présente invention sera décrit ci-après en référence aux figures 1 à 3.

Ce procédé est réalisé sous pression et comprend la solidification de la matière biologique par un fluide cryogénique. La solidification de la matière biologique s'effectue à une température cryogénique, à une pression de 10 bars à 1000 bars et le fluide cryogénique est présent dans une quantité supérieure à 200 kg/m³.

Le procédé comprend en outre avant l'étape de solidification :
- l'introduction de la matière biologique dans une enceinte contenant un fluide cryogénique ; et
- le réglage de conditions souhaitées de température, pression et quantité de fluide cryogénique à l'intérieur de l'enceinte à une température cryogénique, de sorte que la pression régnant à l'intérieur de l'enceinte soit de 10 bars à 1000 bars et que la quantité de fluide cryogénique contenue dans l'enceinte soit supérieure à 200 kg/m³.

Les termes de « matière biologique » doivent être compris dans l'ensemble du présent exposé comme désignant toute matière composée d'au moins une cellule ainsi que tout autre élément apparaissant naturellement dans un organisme biologique. L'identité de l'organisme biologique n'a pas d'importance et dépend de l'application envisagée pour la matière biologique conservée. Notamment l'organisme biologique peut être humain ou non-humain. La cellule comprise dans cette matière est de préférence vivante, c'est-à-dire que la majorité des processus chimiques qui se produisent naturellement dans la cellule surviennent encore. Des exemples de matière biologique sont : un microorganisme unicellulaire, un microorganisme pluricellulaire, une cellule d'un organisme pluricellulaire, tout ou partie d'un tissu ou tout ou partie d'un organe. Parmi les microorganismes unicellulaires, on peut citer les levures (ex : les *Saccharomyces* tels que S. *cerevisiae* et *S. boulardii*), bactéries (ex : les Lactobacilles tel que *L. delbrueckii, notamment L. bulgaricus,* et les Streptocoques tel que *S. thermophilus*) et certaines algues (ex : les diatomées). Parmi les cellules d'un organisme pluricellulaire, on peut citer les cellules souches, les gamètes, certaines algues (ex : les *Arthrospira telles que A. platensis et A. maxima* communément appelés spiruline et utilisés comme complément alimentaire) et les mycètes filamenteux (ex : les *Penicilliums, tels que P. roqueforti et P. camemberti*). Parmi les organes, on peut citer le myocarde, les reins, le pancréas, le foie, un membre (bras, main, jambe, pied), une articulation (coude, genou) et un œil. Parmi les tissus, on peut citer la moelle osseuse et la peau. Des exemples préférés de matière biologique sont : les microorganismes (pathogènes et non-pathogènes), les cellules souches et les organes.

Les termes de « fluide cryogénique » désignent, dans le présent exposé et contrairement à la définition de l'US National Institute of Standards and Technology, un liquide, un gaz ou un composé dans un état supercritique, qui dans l'état en question présente une température inférieure à 170 K, de préférence inférieure à 150 K. De manière générale, les fluides cryogéniques présentent un point d'ébullition très bas, typiquement en dessous de 120 K à pression atmosphérique. Des exemples de fluides cryogéniques sont (le point d'ébullition est précisé entre parenthèses) : l'hélium (5,19 K pour l'hélium 3 ; 4,214 K pour l'hélium 4), l'hydrogène (20,27 K), le néon (27,09 K), l'azote (77,36 K), l'air (78,8 K), l'argon (87,24 K), l'oxygène (90,19 K). Le fluide cryogénique préféré est l'azote pour sa facilité d'accès et d'utilisation. Le fluide cryogénique, quelle que soit sa nature, est utilisé sous forme liquide ou supercritique. Ainsi, les conditions de température, pression et quantité sont de préférence choisies de manière à ce que le fluide cryogénique soit présent à l'intérieur de l'enceinte uniquement à l'état liquide ou uniquement à l'état supercritique.

L'introduction de la matière biologique dans l'enceinte comprenant le fluide cryogénique met au contact celle-ci avec ce dernier. L'introduction est notamment réalisée par immersion de la matière biologique directement dans le fluide cryogénique.

Le réglage de la température, la pression et la quantité de fluide cryogénique doit être compris comme un réglage permettant *in fine* d'obtenir les conditions souhaitées dans l'enceinte comprenant le fluide cryogénique et la matière biologique pendant le refroidissement de celle-ci. Ainsi, la température, la pression et la quantité de fluide cryogénique, avant l'introduction de la matière biologique à l'intérieur de l'enceinte, peuvent différer des température, pression et quantité de fluide cryogénique souhaitées pour la solidification de la matière biologique par le froid.

L'étape de réglage des conditions souhaitées peut survenir avant, pendant ou après l'introduction de la matière biologique dans l'enceinte.

La température est préférentiellement réglée de manière à être inférieure à : 170 K, 150 K, 140 K, 120 K, 100 K, 80 K. De préférence, la température est réglée de manière à être supérieure à 4 K, plus préférentiellement supérieure à 30 K, encore plus préférentiellement supérieure à 70 K.

La pression est réglée de préférence de manière à être supérieure à : 20 bars, 30 bars, 40 bars, 50 bars, 60 bars, 70 bars, 80 bars, 90 bars, ou 100 bars. De préférence, la pression est réglée entre 10 et 1000 bars, 10 et 500 bars, 10 et 250 bars, 10 et 100 bars. Par exemple, la pression peut être 10, 20, 30, 40, 50, 60, 70, 80, 90, 100, 150, 200, 250, 300, 350, 400, 450, 500, 725, 1000 bars.

La quantité de fluide cryogénique contenu dans l'enceinte est réglée de manière à être supérieure à 200 kg/m³, de préférence supérieure à 250 kg/m³, encore de préférence supérieure à 300 kg/m³, encore de préférence supérieure à 350 kg/m³, toujours de préférence supérieure à 400 kg/m³. De préférence, la quantité de fluide cryogénique est réglée de manière à être inférieure à : 850 kg/m³, 825 kg/m³, 800 kg/m³.

Les limites supérieures et inférieures mentionnées ci-dessus pour la température, la pression et la quantité de fluide peuvent être combinées librement selon les besoins.

Le procédé comprend avantageusement en outre une étape de dépressurisation de l'enceinte à une pression atmosphérique. Celle-ci est réalisée de préférence de manière contrôlée pour des raisons de sécurité évidentes ; une libération trop brutale pouvant entrainer jusqu'à un déplacement de l'enceinte et la mise en danger des personnes présentes.

Dans un premier mode de mise en œuvre, la matière biologique est contenue en suspension dans un mélange liquide. Le mélange liquide dans lequel la matière biologique est en suspension peut par exemple être choisi parmi : un milieu de culture, un sérum physiologique, de l'eau ou un produit alimentaire (jus de fruits ou de légumes par exemple). De préférence, le mélange liquide comprend très peu, voire est exempt, d'actifs cryoprotecteurs. C'est-à-dire qu'il comprend moins de 1 % en poids d'actifs cryoprotecteurs, de préférence moins de 0,1 %, encore de préférence moins de 0,01 %, toujours de préférence 0 %.

Dans ce cas, le réglage des conditions souhaitées est préférablement réalisé avant l'introduction de la matière biologique à l'intérieur de l'enceinte. Toujours dans ce cas, l'introduction de la matière biologique est réalisée de préférence par injection du mélange liquide à l'intérieur de l'enceinte.

Le fluide cryogénique est sous forme liquide ou supercritique. L'utilisation du fluide cryogénique sous forme supercritique est particulièrement avantageuse pour la matière biologique (cellules individuelles) en suspension dans un mélange liquide.

Ce premier mode de mise en œuvre conduit généralement à la formation de morceaux solides de mélange comprenant la matière biologique. Ces morceaux solides se présentent généralement sous forme de billes sphériques ou ovoïdes ou de granulés. La géométrie des morceaux solides est généralement fonction de la viscosité du mélange liquide contenant la matière biologique en suspension, de la taille et de la forme des buses d'injection utilisées, ainsi que de la vitesse d'injection.

Par exemple, un produit de viscosité égale à 2 cP traversant des buses de 3 mm de diamètre et de 40 mm de long à une vitesse de 8 mL/min formera des billes sphériques, alors qu'un produit de viscosité égale à 13 000 cP traversant les mêmes buses à une vitesse de 200 mL/min formera des granulés cylindriques. Les viscosités sont mesurées à l'aide d'un viscosimètre à chute de bille.

L'injection peut être réalisée à l'aide d'une pompe à piston ou tout autre mécanisme adapté. La pompe à piston peut comprendre une ou plusieurs buses.

Dans un deuxième mode de mise en œuvre, l'enceinte comprend une première partie d'enceinte et une deuxième partie d'enceinte, les volumes internes des première et deuxième parties d'enceinte étant au départ séparés. En d'autres termes, l'enceinte est formée de deux parties devant être mise en communication fluidique pour la solidification de la matière biologique. Une enceinte en deux parties peut par exemple être constituée d'une cuve cylindrique, de préférence à fond bombé, adaptée aux conditions de pression et de température du procédé innovant, dont l'intérieur est constitué d'un système à double enveloppe. Chaque enveloppe cylindrique est ainsi adaptée aux conditions de traitement du procédé innovant et la mise en commun des deux volumes peut se faire via l'ouverture d'une vanne ou la libération d'un clapet, astucieusement placé à l'interface. Un système équivalent peut être obtenu grâce à deux enceintes reliées par un circuit contenant une vanne dont l'ouverture permet la mise en commun des deux volumes lors de son ouverture.

Dans ce cas, l'étape d'introduction peut comprendre la disposition de la matière biologique dans la première partie d'enceinte exempte de fluide cryogénique, la disposition du fluide cryogénique dans la deuxième partie d'enceinte et la réunion de la première partie d'enceinte avec la deuxième partie d'enceinte pour former l'enceinte de sorte que leurs volumes internes soient en communication fluidique. Toujours dans ce cas, l'étape de réglage des conditions souhaitées peut être réalisée dans la deuxième partie d'enceinte de sorte à obtenir les conditions souhaitées dans l'enceinte après la réunion de la première partie d'enceinte avec la deuxième partie d'enceinte.

La réunion des deux parties d'enceinte est typiquement réalisée de manière rapide et brutale, *i.e.* en l'espace de quelques secondes.

Le deuxième mode de mise en œuvre est particulièrement avantageux pour une matière biologique sous forme solide au début du procédé. Par exemple, la matière biologique est tout ou partie d'un organe ou d'un tissue.

Le procédé de refroidissement décrit ci-dessus fait avantageusement partie d'un procédé de conservation de la matière biologique. Un tel procédé de conservation de la matière biologique comprend alors les étapes du procédé de refroidissement décrites ci-dessus et en outre le transfert de la matière biologique solidifiée dans un congélateur à une température inférieure à 0°C, de préférence inférieure à -10°C, encore de préférence inférieure à -15°C et de préférence au-dessus de -80°C, -60°C et encore de préférence au-dessus de -40°C. La matière biologique solidifiée est notamment extraite de l'enceinte avant son transfert dans un congélateur.

Le produit congelé (hors de la portée des revendications) pouvant être obtenu par le procédé ci-dessus comprend de la matière biologique (humaine ou non-humaine) congelée préalablement traitée et présentant un taux de survie après décongélation de la matière biologique d'au moins 45%, de préférence d'au moins : 50%, 60%, 70%, 80%, 90%, 95%, 98%.

La présente divulgation propose également un procédé de récupération du produit pouvant être obtenu par le procédé de refroidissement décrit ci-dessus. Ce procédé de décongélation comprend les étapes d'introduction de la matière biologique solidifiée dans une enceinte portée à la température de conservation du produit, l'augmentation en pression à l'intérieur de l'enceinte, la décongélation de la matière biologique à l'intérieur de l'enceinte, l'abaissement de la pression à l'intérieur de l'enceinte jusqu'à la pression atmosphérique et la récupération de la matière biologique décongelée.

L'augmentation de la pression peut se réaliser jusqu'à une pression supérieure à : 20 bars, 30 bars, 40 bars, 50 bars, 60 bars, 70 bars, 80 bars, 90 bars, ou 100 bars. De préférence, la pression est réglée entre 10 et 1000 bars, 10 et 500 bars, 10 et 250 bars, 10 et 100 bars. Par exemple, la pression peut être 10, 20, 30, 40, 50, 60, 70, 80, 90, 100, 150, 200, 250, 300, 350, 400, 450, 500, 725, 1000 bars. De préférence, la pression est réglée à la même valeur que celle utilisée lors de la congélation de la matière biologique selon le procédé.

La décongélation peut être réalisée sans apport de chaleur. C'est-à-dire qu'après avoir porté l'enceinte à la température de conservation du produit et l'introduction de la matière biologique à l'intérieur de celle-ci, l'enceinte n'est ni refroidie ni réchauffée. Ainsi, la décongélation a lieu par déplacement à l'équilibre thermique entre l'enceinte et l'environnement de celle-ci.
La décongélation peut être réalisée par apport de chaleur en chauffant à une température supérieure de 10°C à la température de fusion du produit. Alternativement, la décongélation peut être réalisée à un gradient de température supérieur à 1°C/min tant que la température du produit est de plus de 5°C inférieure à sa température de fusion (par exemple 2°C/min, 3°C/min, 4°C/min, 5°C/min, 6°C/min, 7°C/min, 8°C/min, 9°C/min, 10°C/min ou plus), puis inférieure à 1°C/min, jusqu'à la fin de la décongélation (par exemple 0,9°C/min, 0,8°C/min, 0,7°C/min, 0,6°C/min, 0,5°C/min, 0,4°C/min, 0,3°C/min, 0,2°C/min, 0,1°C/min ou moins). Par exemple, pour un produit stocké à -20°C et dont la température de fusion serait de -2°C, la décongélation pourrait s'opérer avec un gradient de 5°C/min entre -20°C et -7°C, puis avec un gradient de 0,5°C/min entre -7°C et 8°C.

### Exemples

### Exemple 1 : conservation sous haute pression en condition liquide

80 mL de lait entier stérilisé (UHT) est ensemencé avec 400.000 ufc/mL de *Lactobacillus bulgaricus* pour former un mélange liquide dont la viscosité est de 4 cP environ.

Un contenant de 2 L adapté au procédé est ensuite rempli avec du diazote liquide avant d'être fermé et mis sous pression de manière à obtenir les conditions suivantes :
- P = 75 bars ;
- T = 80 K ;
- D = 800 kg/m³ de diazote.

L'azote liquide est alors présent dans l'enceinte sous forme liquide uniquement, bien que l'on se situe au-delà de la pression supercritique.

Le mélange liquide est alors injecté par le haut du contenant, au travers d'une buse cylindrique de 3 mm de diamètre et de 10 mm de long, grâce à une pompe à piston, de sorte qu'il s'écoule sous forme d'un goutte-à-goutte rapide à une vitesse d'environ 8 mL/min. Une fois les 80 mL du mélange liquide injectés, la pression est ramenée à la pression atmosphérique et le contenant est ouvert de manière à récupérer un produit cryogénisé sous forme de billes rondes, qui sont réparties dans quatre récipients stériles de 20 mL, lesquels sont rapidement placés dans un congélateur à -20°C. La température de la matière biologique solidifiée est généralement comprise entre -100°C et -80°C au moment où les récipients sont placés au congélateur.

Les quatre récipients sont respectivement ramenés à température ambiante après 24 h, 48 h, 1 semaine (168 h) et 2 semaines (336 h) et deux dénombrements des bactéries sont réalisés par la méthode TEMPO^{®} LAB (solution automatisée et standardisée, commercialisée par BIOMERIEUX, basée sur la méthode du nombre le plus probable et adaptée aux bactéries lactiques). Pour les quatre échantillons, les dénombrements ne sont pas significativement différents et la population moyenne dénombrée est de 375.000 ufc/mL.

Le taux de survie observé est donc d'environ 93,75 %

### Exemple 2 : conservation sous basse pression en condition liquide

La préparation des échantillons est identique à celle de l'exemple 1 ci-dessus.

Un contenant de 2 L adapté est ensuite rempli avec du diazote liquide et mis sous pression de manière à obtenir les conditions suivantes :
- P = 20 bars ;
- T = 80 K ;
- D = 800 kg/m³ de diazote.

L'azote liquide est alors présent dans l'enceinte sous forme liquide uniquement.

La suite du procédé est identique à l'exemple 1. Le produit cryogénisé est récupéré sous forme de billes rondes.

Les quatre récipients sont respectivement ramenés à température ambiante après 24 h, 48 h, 1 semaine (168 h) et 2 semaines (336 h) et deux dénombrements des bactéries sont réalisés par la méthode TEMPO^{®} LAB. Pour les quatre échantillons, les dénombrements ne sont pas significativement différents et la population moyenne dénombrée est d'environ 190.000 ufc/mL.

Le taux de survie observé est donc de 47,5 %.

### Exemple 3 : conservation sous haute pression en condition supercritique

La préparation des échantillons est identique à celle de l'exemple 1 ci-dessus.

Un contenant de 2 L adapté est ensuite rempli avec du diazote liquide et mis sous pression de manière à obtenir les conditions suivantes :
- P = 90 bars ;
- T = 150 K ;
- D = 400 kg/m³ de diazote.

L'azote liquide est alors présent dans l'enceinte sous forme supercritique uniquement.

La suite du procédé est identique à l'exemple 1. Le produit cryogénisé est récupéré sous forme de billes rondes.

Les quatre récipients sont respectivement ramenés à température ambiante après 24 h, 48 h, 1 semaine (168 h) et 2 semaines (336 h) et deux dénombrements des bactéries sont réalisés par la méthode TEMPO^{®} LAB. Pour les quatre échantillons, les dénombrements ne sont pas significativement différents et la population moyenne dénombrée est d'environ 380.000 ufc/mL.

Le taux de survie observé est donc de 95 %.

### Exemple comparatif 1 : congélation classique à -20°C

750 mL de lait entier stérilisé (UHT) est ensemencé avec 400.000 ufc/mL de *Lactobacillus bulgaricus* pour former un mélange liquide.

20 mL du mélange liquide sont versés dans quatre récipients stériles. Les récipients contenant chacun 20 mL du mélange liquide sont ensuite placés dans un congélateur à -20°C.

Les quatre récipients sont respectivement ramenés à température ambiante après 24 h, 48 h, 1 semaine (168 h) et 2 semaines (336 h) et deux dénombrements des bactéries sont réalisés par la méthode TEMPO^{®} LAB. Pour les quatre échantillons, les dénombrements sont négatifs, c'est-à-dire que la population résiduelle revivifiable est inférieure à 1.000 ufc/mL.

Le taux de survie observé est donc, pour les quatre durée de congélation à -20°C, inférieur à 0,25 %

### Exemple comparatif 2 : conservation sous basse pression en conditions vapeur

La préparation des échantillons est identique à celle de l'exemple 1 ci-dessus.

Un contenant de 2 L adapté est ensuite rempli avec du diazote liquide et mis sous pression de manière à obtenir les conditions suivantes :
- P = 20 bars ;
- T = 80 K ;
- D = 80 kg/m³ de diazote.

La suite du procédé est identique à l'exemple 1. Le produit cryogénisé est récupéré sous forme d'un bloc de produit congelé. Le bloc est cassé à l'aide d'une spatule de manière à répartir le produit dans les quatre récipients.

Les quatre récipients sont respectivement ramenés à température ambiante après 24 h, 48 h, 1 semaine (168 h) et 2 semaines (336 h) et deux dénombrements des bactéries sont réalisés par la méthode TEMPO^{®} LAB. Pour les quatre échantillons, les dénombrements ne sont pas significativement différents et la population moyenne dénombrée est de 10.000 ufc/mL.

Le taux de survie observé est donc d'environ 2,5 %.

### Exemple 4 : conservation de spiruline sous haute pression

40 g de spiruline fraiche (*Arthrospira platensis*), sous forme de pâte obtenue après égouttage, sont ajoutés à 40 mL d'une solution d'eau contenant 5 g/L de chlorure de sodium aboutissant à 80 mL de préparation.

Un réservoir de 2 L adapté au procédé est ensuite rempli avec du diazote liquide et mis sous pression de manière à obtenir les conditions suivantes :
- P = 60 bars ;
- T = 140 K ;
- D = 800 kg/m³ de diazote.

**L'azote** est alors présent dans l'enceinte sous forme liquide.

La préparation est alors injectée par le haut du récipient grâce à une pompe à piston, de telle sorte qu'elle s'écoule sous forme d'un goutte à goutte rapide. Une fois les 80 mL de préparation injectés, la pression est ramenée à la pression atmosphérique et le réservoir est ouvert de manière à récupérer le produit cryogénisé, qui est réparti dans quatre récipients stériles de 20 mL, lesquels sont rapidement placés dans un congélateur à -20°C. La température des produits est généralement comprise entre -100°C et -80°C au moment où les récipients sont placés au congélateur.

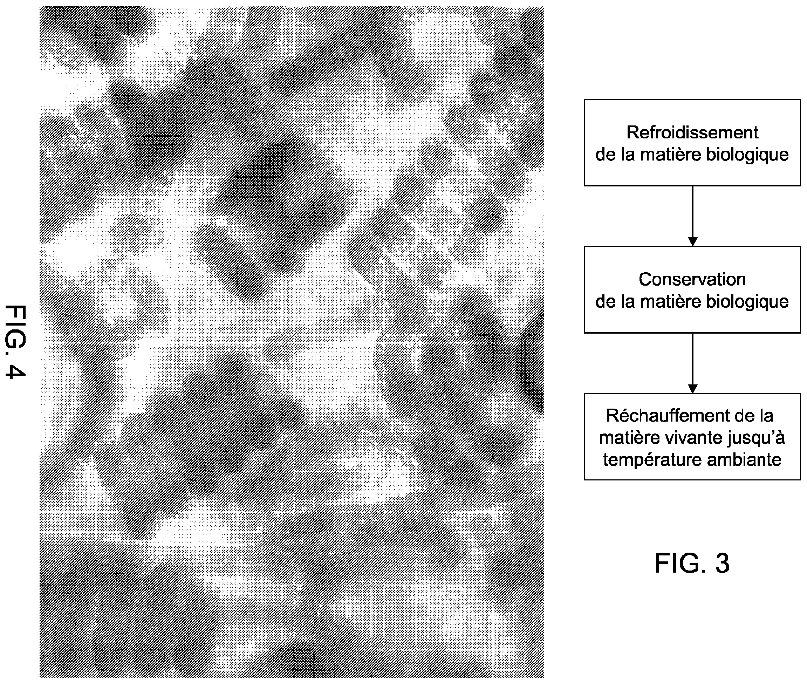

Un prélèvement est réalisé dans chacun des quatre récipients après respectivement 24 h, 48 h, 1 semaine (168 h) et 2 semaines (336 h). Les prélèvements sont ramenés à température ambiante est observés au microscope. Aucune altération particulière n'est observée sur les quatre échantillons. La figure 4 présente une photographie de la dernière observation réalisée.

## Revendications

1. Procédé de refroidissement de matière biologique par cryogénie sous haute pression, comprenant la solidification de la matière biologique par un fluide cryogénique, dans lequel la solidification de la matière biologique s'effectue à une température cryogénique, à une pression de 10 bars à 1000 bars, le fluide cryogénique est présent dans une quantité supérieure à 200 kg/m³ et le fluide cryogénique est dans l'état liquide ou supercritique,
comprenant en outre avant l'étape de solidification :
- l'introduction de la matière biologique dans une enceinte contenant un fluide cryogénique par immersion de la matière biologique directement dans le fluide cryogénique ; et
- le réglage de conditions souhaitées de température, pression et quantité de fluide cryogénique à l'intérieur de l'enceinte à une température cryogénique, de sorte que la pression régnant à l'intérieur de l'enceinte soit de 10 bars à 1000 bars et que la quantité de fluide cryogénique contenue dans l'enceinte soit supérieure à 200 kg/m³ .

2. Procédé selon la revendication 1, dans lequel la pression est de 10 à 500 bars.

3. Procédé selon la revendication 2, dans lequel la pression est de 10 à 100 bars.

4. Procédé selon l'une quelconque des revendications précédentes, dans lequel la température est réglée de manière à être inférieure à 170 K.

5. Procédé selon l'une quelconque des revendications précédentes, dans lequel la pression est réglée de manière à être supérieure à 20 bars.

6. Procédé selon l'une quelconque des revendications précédentes, dans lequel la quantité de fluide cryogénique contenu dans l'enceinte est réglée de manière à être supérieure à 250 kg/m³.

7. Procédé selon l'une quelconque des revendications précédentes dans lequel la matière biologique est au préalable contenue en suspension dans un mélange liquide ; le réglage de la température cryogénique, de la pression de 10 bars à 1000 bars et de la quantité de fluide cryogénique est réalisé avant l'introduction de la matière biologique à l'intérieur de l'enceinte ; et dans lequel l'introduction de la matière biologique est réalisée par injection du mélange liquide à l'intérieur de l'enceinte.

8. Procédé selon l'une quelconque des revendications précédentes, dans lequel l'enceinte comprend une première partie d'enceinte et une deuxième partie d'enceinte, les volumes internes des première et deuxième parties d'enceinte étant au départ séparés ;
l'étape d'introduction comprenant la disposition de la matière biologique dans la première partie d'enceinte exempte de fluide cryogénique, la disposition du fluide cryogénique disposé dans la deuxième partie d'enceinte et la réunion de la première partie d'enceinte avec la deuxième partie d'enceinte pour former l'enceinte de sorte que leurs volumes internes soient liés ;
le réglage de la température cryogénique, de la pression de 10 bars à 1000 bars et de la quantité de fluide cryogénique étant réalisé dans la deuxième partie d'enceinte de sorte à obtenir les conditions souhaitées dans l'enceinte après la réunion de la première partie d'enceinte avec la deuxième partie d'enceinte.

9. Procédé de conservation de matière biologique par cryogénie sous haute pression, comprenant les étapes du procédé selon l'une des revendications 1 à 8, la dépressurisation de l'enceinte à la pression atmosphérique et la disposition de la matière biologique solidifiée dans un congélateur.

10. Procédé de conservation de matière biologique par cryogénie sous haute pression, comprenant les étapes du procédé selon l'une des revendications 1 à 9 dans lequel la matière biologique est non humaine.

## Patentansprüche

1. Verfahren zur Kühlung von biologischem Material durch Kryogenik unter hohem Druck, das die Erstarrung des biologischen Materials durch ein kryogenes Fluid umfasst, wobei die Erstarrung des biologischen Materials bei kryogener Temperatur und einem Druck von 10 bar bis 1000 bar erfolgt und das kryogene Fluid in einer Menge von mehr als 200 kg/m3 in flüssigem oder überkritischem Zustand vorhanden ist, und das ferner vor dem Schritt der Erstarrung folgendes umfasst:
- das Einbringen des biologischen Materials in einen Behälter mit kryogenem Fluid durch direktes Eintauchen des biologischen Materials in das kryogene Fluid; und
- das Einstellen gewünschter Bedingungen hinsichtlich Temperatur, Druck und Menge des kryogenen Fluids im Innern des Behälters, wobei die Temperatur kryogen ist, der Druck im Behälter zwischen 10 bar und 1000 bar liegt und die Menge des im Behälter enthaltenen kryogenen Fluids größer als 200 kg/m³ ist.

2. Verfahren nach Anspruch 1, in dem der Druck 10 bis 500 bar beträgt.

3. Verfahren nach Anspruch 2, in dem der Druck 10 bis 100 bar beträgt.

4. Verfahren nach einem der vorhergehenden Ansprüche, in dem die Temperatur so eingestellt ist, dass sie unter 170 K liegt.

5. Verfahren nach einem der vorhergehenden Ansprüche, in dem der Druck so eingestellt ist, dass er größer als 20 bar ist

6. Verfahren nach einem der vorhergehenden Ansprüche, in dem die Menge des in der Kammer enthaltenen kryogenen Fluids so eingestellt ist, dass sie größer als 250 kg/m3 ist.

7. Verfahren nach einem der vorhergehenden Ansprüche, in dem das biologische Material zuvor in einer Flüssigmischung suspendiert ist; die Einstellung der kryogenen Temperatur, des Drucks von 10 bar bis 1000 bar und der Menge des kryogenen Fluids erfolgt vor der Einbringung des biologischen Materials in die Kammer, und die Einbringung des biologischen Materials erfolgt durch Einspritzung der Flüssigmischung in die Kammer.

8. Verfahren nach einem der vorhergehenden Ansprüche, in dem die Kammer einen ersten Teil der Kammer und einen zweiten Teil der Kammer umfasst, wobei die Innenvolumina des ersten und zweiten Teils der Kammer anfangs getrennt sind; der Schritt des Einbringens umfasst das Platzieren des biologischen Materials in den ersten Teil der Kammer, der frei von kryogenem Fluid ist, das Platzieren des kryogenen Fluids in den zweiten Teil der Kammer sowie die Verbindung des ersten Teils der Kammer mit dem zweiten Teil, um eine Kammer zu bilden, in der die Innenvolumina zu einem einzigen Innenvolumen zusammengeführt sind;
wobei die Einstellung der kryogenen Temperatur, des Drucks von 10 bar bis 1000 bar und der Menge des kryogenen Fluids im zweiten Teil der Kammer erfolgt, sodass nach dem Verbinden des ersten Teils der Kammer mit dem zweiten die gewünschten Bedingungen in der Kammer erzielt werden.

9. Verfahren zur Konservierung von biologischem Material durch Kryogenik unter hohem Druck, das die Verfahrensschritte nach einem der Ansprüche 1 bis 8, die Druckentlastung der Kammer auf Umgebungsdruck und das Platzieren des verfestigten biologischen Materials in einen Tiefkühlschrank.

10. Verfahren zur Konservierung von biologischem Material durch Kryogenik unter hohem Druck, das die Verfahrensschritte nach einem der Ansprüche 1 bis 9 umfasst, wobei das biologische Material nicht menschlich ist.

## Claims

1. A high pressure cryogenic process for cooling a biological material, comprising the solidification of the biological material by a cryogenic fluid, wherein the solidification of the biological material is carried out at a cryogenic temperature at a pressure from 10 to 1,000 bars the cryogenic fluid is present in an amount greater than 200kg/m3, and the cryogenic fluid is in liquid or supercritical form, comprising before the solidification step:
- the introduction of the biological material into a chamber containing a cryogenic fluid; and
- the setting of the desired conditions of temperature, pressure and quantity of cryogenic fluid within the chamber to a cryogenic temperature, so that the pressure inside the chamber is between 10 bars and 1000 bars and the quantity of cryogenic fluid in the chamber is greater than 200kg/m3.

2. The process according to claim 1, wherein the pressure ranges from 10 to 500 bars.

3. The process according to claim 2, wherein the pressure ranges from 10 to 100 bars.

4. The process according to any of the previous claims, wherein the temperature is controlled so as to be lower than 170K.

5. The process according to any of the previous claims, wherein the pressure is set be greater than 20 bars.

6. The process according to any of the previous claims, wherein the quantity of cryogenic fluid contained in the chamber is adjusted so as to be greater than 250kg/m3.

7. Process according to any of the previous claims, wherein the biological material is first contained in suspension in a liquid mixture; the setting of the cryogenic temperature, of the pressure between 10 bars and 1000 bars and the quantity of cryogenic fluid is performed prior to the introduction of the biological material into the chamber; and wherein the introduction of the biological material is performed by injection of the liquid mixture into the chamber.

8. Process according to any of the previous claims, wherein the chamber comprises a first chamber portion and a second chamber portion, the internal volumes of the first and second chamber portions being separated at the beginning;
the step of introduction comprising the placing of the biological material in the first chamber portion free of cryogenic fluid, the placing of the cryogenic fluid in the second chamber portion and the connection of the first chamber portion with the second chamber portion to form the chamber so that the internal volumes thereof are connected; and
setting of the cryogenic temperature, of the pressure between 10 bars and 1000 bars and the quantity of cryogenic fluid being provided in the second chamber portion so as to achieve the desired conditions within the chamber after connecting the first chamber portion with the second chamber portion.

9. Process for the high pressure preservation of a biological material, comprising the steps of the process according to any of claims 1 to 8, the depressurization of the chamber to the atmospheric pressure and the placing of the solidified biological material in a freezer.

10. Process for the high pressure preservation of a biological material, comprising the steps of the process according to any of claims 1 to 9, wherein the biological material is not human.
